(19) Europäisches Patentamt
European Patent Office
Office européen des brevets

(11) **EP 1 983 337 A2**

(12) **EUROPEAN PATENT APPLICATION**

(43) Date of publication:
**22.10.2008 Bulletin 2008/43**

(51) Int Cl.:
*G01N 25/18* (2006.01)      *G01N 33/00* (2006.01)

(21) Application number: **08250359.0**

(22) Date of filing: **30.01.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **30.01.2007 JP 2007020055**

(71) Applicant: **Mitsui Mining and Smelting Co., Ltd.**
**Shinagawa-ku,**
**Tokyo 141-8584 (JP)**

(72) Inventors:
• **Koike, Atsushi**
**Ageo-Shi**
**Saitama 362-0021 (JP)**
• **Tsuruda, Mayuko**
**Ageo-Shi**
**Saitama 362-0021 (JP)**

(74) Representative: **Hall, Matthew Benjamin**
**Frank B. Dehn & Co.**
**St Bride's House**
**10 Salisbury Square**
**London**
**EC4Y 8JD (GB)**

(54) **Method and apparatus for measuring amount of generated ammonia**

(57)    Methods and apparatuses are provided for measuring an amount of generated ammonia from a urea concentration and an ammonium formate concentration in a mixture solution of an aqueous urea solution and an ammonium formate solution.

The method for measuring an amount of ammonia generated from a sample solution being an aqueous urea solution, an aqueous ammonium formate solution, or an aqueous mixture thereof, includes: applying a pulse voltage to a heating element for a predetermined time to heat the sample solution using the heating element; measuring a thermal conductivity-dependent output value and a kinetic viscosity-dependent output value that are electrical outputs dependent on electric resistivity of a temperature-sensitive element; calculating a urea concentration X wt% and an ammonium formate concentration Y wt% in the sample solution from a relationship between the thermal conductivity-dependent output value and the kinetic viscosity-dependent output value; calculating a urea amount A and an ammonium formate amount B in the sample solution from their concentrations and an amount of the sample solution; and determining the amount of generated ammonia with data thus obtained.

[Fig.6]

**Description**

The present invention relates to a method for measuring an amount of ammonia generated from a sample solution being an aqueous urea solution, an aqueous ammonium formate solution, or an aqueous mixture thereof; and an apparatus for measuring an amount of generated ammonia.

In an internal combustion engine of an automobile, fossil fuels such as gasoline and light fuel oil are burnt. The consequent exhaust gases contain environmental pollutants such as unburned carbon monoxide (CO), hydrocarbons (HC), sulfur oxides (SOx) and nitrogen oxides (NOx), along with water, carbon dioxide and the like. In recent years, especially in order to protect the environment and prevent pollution of the living environment, various measures to purify the automobile exhaust gases have been taken.

As one of such measures, the use of a catalytic device for cleaning exhaust gases may be mentioned. In the device, a three-way catalyst for exhaust gas purification is installed in the exhaust system and decomposes CO, HC, NOx and the like by oxidation/reduction reactions, making them harmless. In order to maintain the decomposition of NOx sustainably in the catalytic device, an aqueous urea solution is sprayed to the catalyst from an immediate upstream of the catalytic device in the exhaust system.

In the meanwhile, as mentioned above, in the catalytic device for cleaning exhaust gases, in order to maintain the decomposition of NOx sustainably in the catalytic device an aqueous urea solution is sprayed to the catalyst from an immediate upstream of the catalytic device in the exhaust system. In particular, the use with a urea concentration of 32.5% is most suitable.

However, in this case a solidifying temperature of an aqueous urea solution is relatively high, so that the aqueous urea solution with a concentration of 32.5% solidifies at -11°C. Therefore, the decomposition of NOx in the above-mentioned catalytic device for cleaning exhaust gases is not sustained in extremely cold places, for instance, in Wakkanai in Japan, Alaska, neighboring area of the Great Lakes, Canada, Russia and the like.

For this reason, a mixture solution of an aqueous urea solution and an ammonium formate solution is used instead of an aqueous urea solution itself in the catalytic device for cleaning exhaust gases in such countries as the United States to lower the solidifying temperature, namely the freezing temperature.

In the meanwhile, in order that such mixture solution will not solidify and will catalyze reduction reaction efficiently at an upstream of the catalytic device, it is preferable to set the concentrations of urea, ammonium formate and $H_2O$ at 20 wt%, 26 wt% and 54 wt%, respectively. However, there have not been developed any measures to figure out such suitable mixture ratio.

Therefore, it is an object of the present invention that apparatuses and methods for measuring an amount of generated ammonia are provided whereby when a mixture solution of an aqueous urea solution and an ammonium formate solution is used instead of an aqueous urea solution itself in a catalytic device for cleaning exhaust gases, the concentrations of the mixture solution in a urea tank and the amount of generated ammonia can be precisely and quickly figured out so that the concentrations of the mixture solution can be maintained at a prescribed level and NOx in the exhaust gases can be reduced to a markedly low level.

Methods and apparatuses are provided whereby an amount of ammonia generated when a mixture solution containing an aqueous urea solution and an ammonium formate solution is sprayed to exhaust gases, is measured from the concentrations of urea and ammonium formate in the mixture solution.

The present invention is done to solve the above-mentioned problems in the conventional art and to accomplish the object. A method for measuring an amount of generated ammonia of the present invention is a method for measuring an amount of ammonia generated from a sample solution being an aqueous urea solution, an aqueous ammonium formate solution, or an aqueous mixture thereof, comprising:

    providing a sensor comprising a heating element and a temperature-sensitive element placed near the heating element;
    applying a pulse voltage to the heating element for a predetermined time to heat the sample solution using the heating element;
    measuring a thermal conductivity-dependent output value that is an electrical output from the temperature-sensitive element and depends on thermal conductivity of the sample solution, and a kinetic viscosity-dependent output value that is an electrical output from the temperature-sensitive element and depends on kinetic viscosity of the sample solution, the electrical outputs being dependent on electric resistivity of the temperature-sensitive element;
    calculating a urea concentration X wt% and an ammonium formate concentration Y wt% in the sample solution from a relationship between the thermal conductivity-dependent output value and the kinetic viscosity-dependent output value;
    determining the amount of generated ammonia per unit weight of the sample solution by using the following equation calculated from an amount of generated ammonia A per unit weight of the urea and an amount of generated ammonia B per unit weight of the ammonium formate and their concentrations:

$$\text{the amount of generated ammonia} = X \ / \ 100 \ x \ A \ + \ Y \ / \ 100$$

$$x \ B.$$

An apparatus for measuring an amount of generated ammonia of the present invention is an apparatus for measuring an amount of ammonia generated from a sample solution being an aqueous urea solution, an aqueous ammonium formate solution, or an aqueous mixture thereof, comprising:

a sensor comprising a heating element and a temperature-sensitive element placed near the heating element; and being configured to:

apply a pulse voltage to the heating element for a predetermined time to heat the sample solution using the heating element;
measure a thermal conductivity-dependent output value that is an electrical output from the temperature-sensitive element and depends on thermal conductivity of the sample solution, and a kinetic viscosity-dependent output value that is an electrical output from the temperature-sensitive element and depends on kinetic viscosity of the sample solution, the electrical outputs being dependent on electric resistivity of the temperature-sensitive element;
calculate a urea concentration X wt% and an ammonium formate concentration Y wt% in the sample solution from a relationship between the thermal conductivity-dependent output value and the kinetic viscosity-dependent output value;
determine the amount of generated ammonia per unit weight of the sample solution by using the following equation calculated from an amount of generated ammonia A per unit weight of the urea and an amount of generated ammonia B per unit weight of the ammonium formate and their concentrations:

$$\text{the amount of generated ammonia} = X \ / \ 100 \ x \ A \ + \ Y \ / \ 100$$

$$x \ B.$$

In the method for measuring an amount of generated ammonia and the apparatus for measuring an amount of generated ammonia of the present invention, the urea concentration X wt% and the ammonium formate concentration Y wt% in the sample solution are calculated from the thermal conductivity-dependent output value and the kinetic viscosity-dependent output value of the sample solution that are obtained based on previously memorized calibration curve data that indicate correlations between a thermal conductivity-dependent output value and a kinetic viscosity-dependent output value of predetermined reference solutions.

In the method for measuring an amount of generated ammonia and the apparatus for measuring an amount of generated ammonia of the present invention, the urea concentration X wt% and the ammonium formate concentration Y wt% are obtained by determining at least two tentative kinetic viscosity-dependent output values to which the thermal conductivity-dependent output value of the sample solution corresponds, based on at least two pieces of the calibration curve data; and performing calculation on a pro-rata basis using the at least two tentative kinetic viscosity-dependent output values to determine the urea concentration and the ammonium formate concentration at which the kinetic viscosity-dependent output value is obtained.

In the method for measuring an amount of generated ammonia and the apparatus for measuring an amount of generated ammonia of the present invention,

the sample solution has a urea concentration of X wt% and an ammonium formate concentration of Y wt% and gives a thermal conductivity-dependent output value of V01 and a kinetic viscosity-dependent output value of V02;
first calibration curve data are provided in which a ratio of a urea concentration Xb wt% to an ammonium formate concentration Yb wt%, namely cb = Yb/Xb, is constant;
second calibration curve data are provided in which a ratio of a urea concentration Xc wt% to an ammonium formate concentration Yc wt%, namely cc = Yc/Xc, is constant and larger than the ratio cb;
a first tentative kinetic viscosity-dependent output value V02b and a second tentative kinetic viscosity-dependent output value V02c are obtained to which the thermal conductivity-dependent output value V01 corresponds, based on the first and second calibration curve data;

a ratio of the urea concentration X wt% to the ammonium formate concentration Y wt%, namely c = Y/X, is calculated from the formula: c = cc (V02-V02b) /V02c in which V02b is the first tentative kinetic viscosity-dependent output value and V02c is the second tentative kinetic viscosity-dependent output value; and

the urea concentration X wt% and the ammonium formate concentration Y wt% are calculated based on calibration curve data corresponding to Y/X = c that is selected from among the previously memorized calibration curve data.

In the method for measuring an amount of generated ammonia and the apparatus for measuring an amount of generated ammonia of the present invention, the kinetic viscosity-dependent output value is an electrical output from the temperature-sensitive element that is measured from initiation of the application of the pulse voltage to the passage of a second period and the second period is equal to a period of the application of the pulse voltage.

In the method for measuring an amount of generated ammonia and the apparatus for measuring an amount of generated ammonia of the present invention, the period of the application of the pulse voltage is 5 to 30 seconds.

A method for measuring an amount of generated ammonia of the present invention is a method for measuring an amount of ammonia generated from a sample solution being an aqueous urea solution, an aqueous ammonium formate solution, or an aqueous mixture thereof, comprising:

providing a sensor comprising a heating element and a temperature-sensitive element placed near the heating element;

applying a pulse voltage to the heating element for a predetermined time to heat the sample solution using the heating element;

measuring a thermal conductivity-dependent output value that is an electrical output from the temperature-sensitive element and depends on thermal conductivity of the sample solution, the electrical output being dependent on electric resistivity of the temperature-sensitive element, and measuring a density-dependent output value that is an electrical output dependent on density of the sample solution with a differential pressure sensor;

calculating a urea concentration X wt% and an ammonium formate concentration Y wt% in the sample solution from a relationship between the thermal conductivity-dependent output value and the density-dependent output value;

determining the amount of generated ammonia per unit weight of the sample solution by using the following equation calculated from an amount of generated ammonia A per unit weight of the urea and an amount of generated ammonia B per unit weight of the ammonium formate and their concentrations:

$$\texttt{the amount of generated ammonia = X / 100 x A + Y / 100}$$

$$\texttt{x B.}$$

An apparatus for measuring an amount of generated ammonia of the present invention is an apparatus for measuring an amount of ammonia generated from a sample solution being an aqueous urea solution, an aqueous ammonium formate solution, or an aqueous mixture thereof, comprising:

a sensor comprising a heating element and a temperature-sensitive element placed near the heating element; and being configured to:

apply a pulse voltage to the heating element for a predetermined time to heat the sample solution using the heating element;

measure a thermal conductivity-dependent output value with the sensor, which output value is an electrical output from the temperature-sensitive element and depends on thermal conductivity of the sample solution, the electrical output being dependent on electric resistivity of the temperature-sensitive element, and measure a density-dependent output value with a differential pressure sensor, which output value is an electrical output dependent on density of the sample solution;

calculate a urea concentration X wt% and an ammonium formate concentration Y wt% in the sample solution from a relationship between the thermal conductivity-dependent output value and the density-dependent output value;

determine the amount of generated ammonia per unit weight of the sample solution by using the following equation calculated from an amount of generated ammonia A per unit weight of the urea and an amount of generated ammonia B per unit weight of the ammonium formate and their concentrations:

```
the amount of generated ammonia = X / 100 x A + Y / 100
x B.
```

In the method for measuring an amount of generated ammonia and the apparatus for measuring an amount of generated ammonia of the present invention, the urea concentration X wt% and the ammonium formate concentration Y wt% in the sample solution are calculated from the thermal conductivity-dependent output value and the density-dependent output value of the sample solution that are obtained based on previously memorized calibration curve data that indicate correlations between a thermal conductivity-dependent output value and a density-dependent output value of predetermined reference solutions.

In the method for measuring an amount of generated ammonia and the apparatus for measuring an amount of generated ammonia of the present invention, the urea concentration X wt% and the ammonium formate concentration Y wt% are obtained by determining at least two tentative density-dependent output values to which the thermal conductivity-dependent output value of the sample solution corresponds, based on at least two pieces of the calibration curve data; and performing calculation on a pro-rata basis using the at least two tentative density-dependent output values to determine the urea concentration and the ammonium formate concentration at which the density-dependent output value of the sample solution is obtained.

In the method for measuring an amount of generated ammonia and the apparatus for measuring an amount of generated ammonia of the present invention,

the sample solution has a urea concentration of X wt% and an ammonium formate concentration of Y wt% and gives a thermal conductivity-dependent output value of V01 and a density-dependent output value of V03;

third calibration curve data are provided in which a ratio of a urea concentration Xb wt% to an ammonium formate concentration Yb wt%, namely cb = Yb/Xb, is constant;

fourth calibration curve data are provided in which a ratio of a urea concentration Xc wt% to an ammonium formate concentration Yc wt%, namely cc = Yc/Xc, is constant and larger than the ratio cb;

a first tentative density-dependent output value V03b and a second tentative density-dependent output value V03c are obtained to which the thermal conductivity-dependent output value V01 corresponds, based on the third and fourth calibration curve data;

a ratio of the urea concentration X wt% to the ammonium formate concentration Y wt%, namely c = Y/X, is calculated from the formula: c = cc(V03-V03b)/V03c in which V03b is the first tentative density-dependent output value and V03c is the second tentative density-dependent output value; and

the urea concentration X wt% and the ammonium formate concentration Y wt% are calculated based on calibration curve data corresponding to Y/X = c that is selected from among the previously memorized calibration curve data.

In the methods for measuring an amount of generated ammonia and the apparatuses for measuring an amount of generated ammonia of the present invention, the thermal conductivity-dependent output value is an electrical output from the temperature-sensitive element that is measured from initiation of the application of the pulse voltage to the passage of a first period and the first period is 1/2 or less of the period of the application of the pulse voltage.

In the methods for measuring an amount of generated ammonia and the apparatuses for measuring an amount of generated ammonia of the present invention, the first period is 0.5 to 3 seconds.

In the methods for measuring an amount of generated ammonia and the apparatuses for measuring an amount of generated ammonia of the present invention, at least one of the previously memorized predetermined reference solutions is an aqueous urea solution having an ammonium formate concentration of 0%.

According to the present invention, an amount of ammonia generated from a sample solution can be measured from an electrical output dependent on the thermal conductivity and an electrical output dependent on the kinetic viscosity of the sample solution.

Certain preferred embodiments of the present invention will now be described, by way of example only, with reference to the accompanying drawings, in which:

Figure 1 shows a schematic cross-sectional diagram of an embodiment of an apparatus for measuring the amount of generated ammonia according to the present invention.

Figure 2 is a schematic cross-sectional diagram of an identification sensor module of the liquid identification apparatus in Fig.1.

Figure 3 is a schematic cross-sectional diagram of a detecting unit for identification of a liquid kind of the liquid identification apparatus in Fig.1.

Figure 4 is a schematic cross-sectional diagram of the liquid identification apparatus in Fig.1 during operation.

Figure 5 is an exploded perspective view of a thin film chip of an indirectly-heated liquid detecting unit.

Figure 6 is a circuit diagram for the identification of a liquid kind.

Figure 7 is a figure showing a relationship between a single pulse voltage P applied to a heating element and a sensor output Q.

Figure 8 shows examples of first and second calibration curves.

Figure 9 is a schematic cross-sectional diagram of another embodiment of the apparatus for measuring the amount of generated ammonia according to the present invention.

Figure 10 shows examples of third and fourth calibration curves.

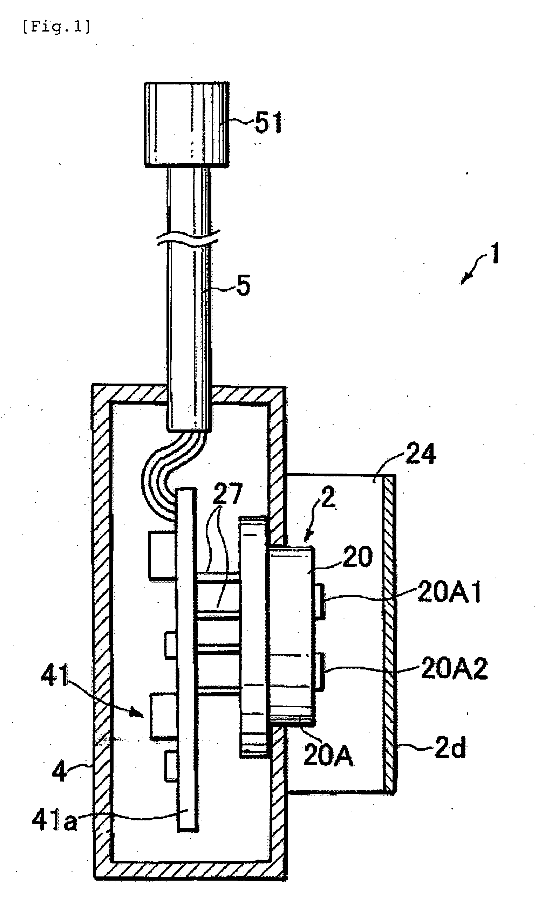

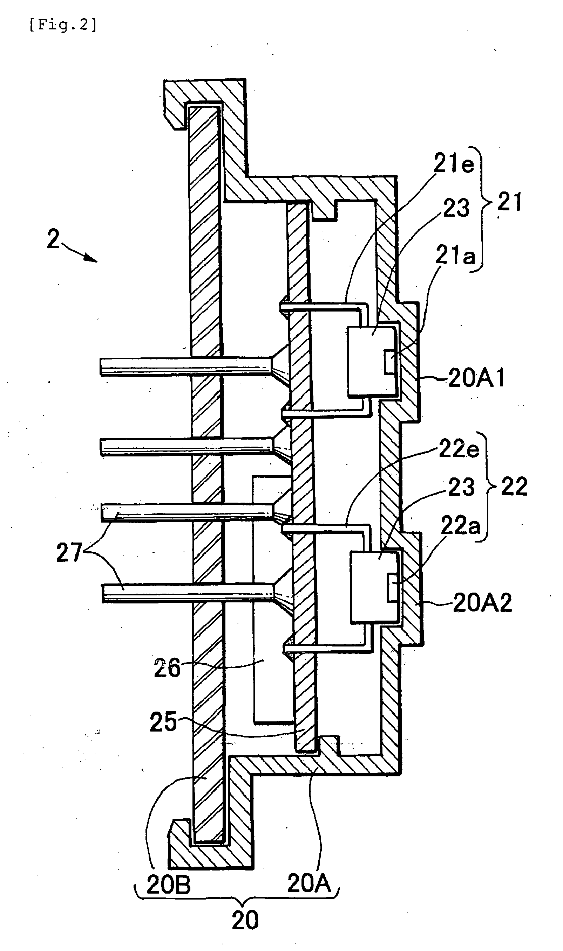

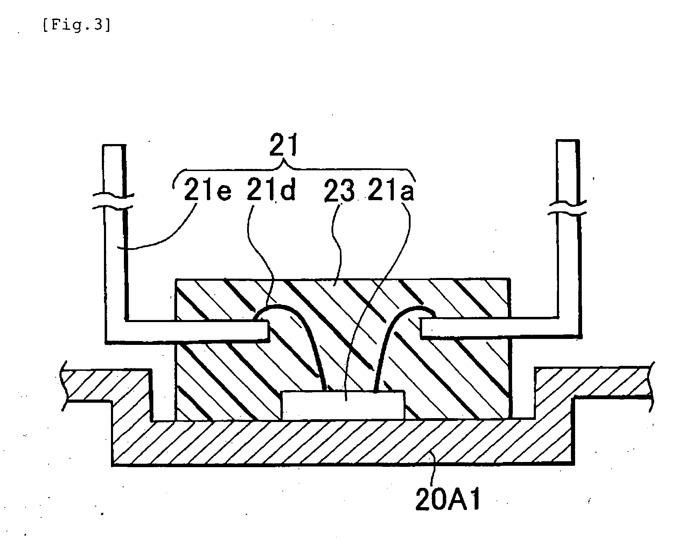

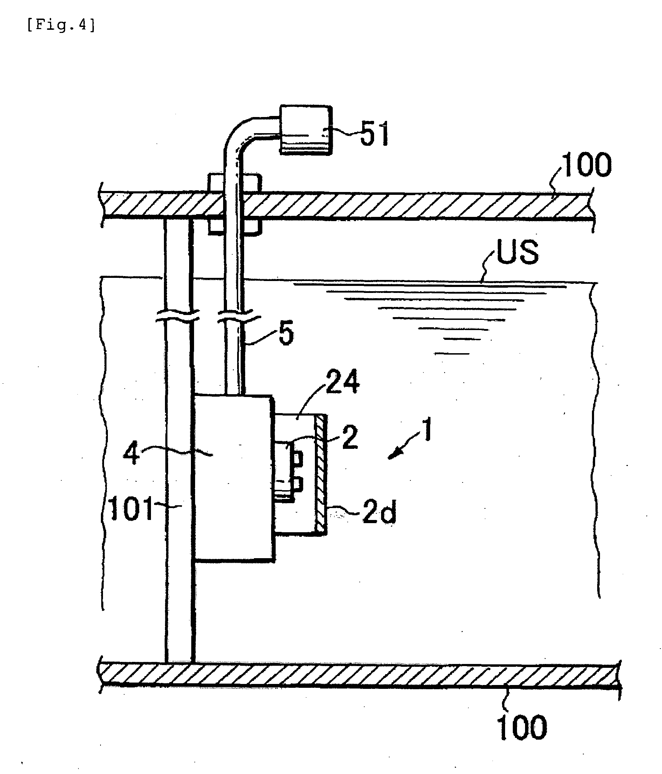

Fig.1 is a schematic cross-sectional diagram showing an embodiment of an apparatus for measuring the amount of generated ammonia according to the present invention, Fig. 2 is a schematic cross-sectional diagram of a sensor module attached to the apparatus for the measurement of the amount of generated ammonia and Fig.3 is a schematic cross-sectional diagram showing a liquid detection unit of the sensor module. Also, Fig.4 is a schematic cross-sectional diagram of the apparatus for measuring the amount of generated ammonia of the present embodiment during operation.

As shown in Fig. 4, an apparatus 1 for measuring the amount of generated ammonia is mounted, for example, on a wall material 101 constituting a dosing pipe unit part placed inside a tank 100 that composes an exhaust gas cleaning system mounted in an automobile and contains a mixture solution of an aqueous urea solution and ammonium formate solution used for the decomposition of NOx. The mounting can be done with screws or by band strapping. As shown in Fig.1 and Fig. 4, the apparatus 1 for measuring the amount of generated ammonia is equipped with a sensor module 2 for the measurement of the amount of generated ammonia, a waterproof case 4 and a waterproof wire 5.

As shown in Fig. 2, the sensor module 2 for the measurement of the generated ammonia contains an indirectly-heated liquid detecting unit 21, a liquid temperature detecting unit 22, a liquid detecting circuit board 25, and application specific IC (ASIC) 26 inside a vessel 20. The vessel 20 is composed of, for example, a main body 20A which is integrally made of a corrosion-resistant material, for example, a metal such as stainless steel, and a lid 20B which is integrally made of a metal such as stainless steel. The main body and the lid are bound together for example by swaging. As shown in Fig. 1, the bound part of the main body 20A and the lid 20B of the vessel is located inside the waterproof case 4.

As shown in Fig.2, two blister parts 20A1 and 20A2 are formed at the bottom part (the right side part in Figs. 1 and 2, and the bottom side part in Fig.3) of the vessel main body 20A, and the liquid detecting unit 21 and the liquid temperature detecting unit 22 are installed with a certain distance therebetween in the up and down directions in the dented parts inside the vessel corresponding to the blister parts. As shown in Fig.3, the liquid detecting unit 21 is made by embedding a liquid detecting thin film chip 21a in a synthetic resin mold 23 in such a way as to expose one side of the chip. The chip is a structure in which a thin film of liquid detecting temperature-sensitive element is formed on a chip substrate as mentioned below. The synthetic resin mold 23 is made of, for instance, an epoxy resin. The exposed side of the thin film chip 21a of the liquid detecting unit (the right side in Fig.2 and the lower side in Fig.3) is in contact with the inside of the dent of the vessel main body 20A.

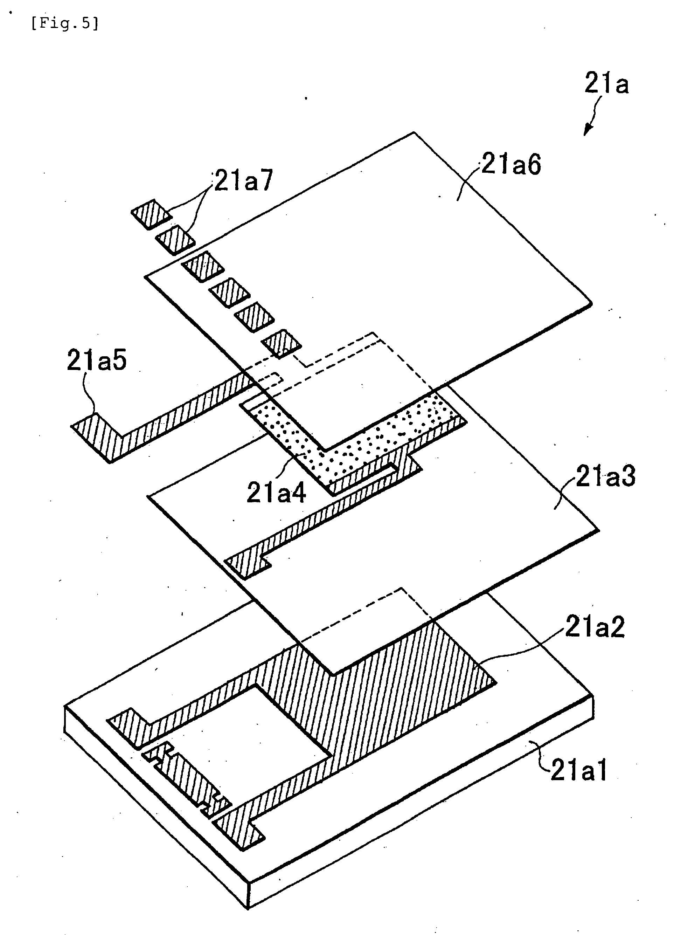

Fig.5 is an exploded perspective view of the liquid detecting thin film chip 21a of the indirectly-heated liquid detecting unit 21. The liquid detecting thin film chip 21a is composed of, for instance, a chip substrate 21al made of $Al_2O_3$, a liquid detecting temperature-sensitive element 21a2 made of Pt, an interlayer insulating film 21a3 made of $SiO_2$, a heating element 21a4 made of $TaSiO_2$, a heating element electrode 21a5 made of Ni, a protection film 21a6 made of $SiO_2$, and an electrode pad 21a7 made of Ti/Au, which are appropriately laminated in sequence. The liquid detecting temperature-sensitive element 21a2 is formed in a serpentine pattern though not shown as such in the figure.

The electrode pad 21a7 connected with the liquid detecting temperature-sensitive element 21a2 and the heating element electrode 21a5, is, as shown in Fig. 3, connected with an outer electrode terminal 21e via a bonding wire 21d.

In the meanwhile, the liquid temperature detecting unit 22 can be composed in a similar manner to that of the liquid detecting unit 21, except that a heating element is not activated and only a temperature-sensitive element (a temperature-sensitive element for liquid temperature detection in the liquid temperature detecting unit 22) is activated. In Fig. 2, an outer electrode terminal of the liquid temperature detecting unit 22 is represented by numeral 22e. In the meanwhile, the liquid temperature detecting unit 22 may be free of a heating element unlike the liquid detecting unit.

As shown in Fig. 2, the outer electrode terminal 21e of the liquid detecting unit 21 and the outer electrode terminal 22e of the liquid temperature detecting unit 22 are connected to a circuit of the liquid detecting circuit board 25. On the liquid detecting circuit board 25, a terminal pin 27 is attached. The terminal pin 27 extends through the vessel lid 20B to the outside of the vessel.

As shown in Fig.1, an electric source circuit unit 41 is installed in the waterproof case 4, and the electric source circuit unit 41 is supported by a supporting means not shown in the figure. The electric source circuit unit 41 is composed of an appropriate circuit element mounted on a circuit board 41a. The electric source circuit unit receives a direct current voltage of, for example, 24V from an outer electric source and, based on the supplied voltage, produces a direct current voltage of, for example, 5V that may be suitable for driving each circuit element of the apparatus 1 for measuring the amount of generated ammonia. On the circuit of the circuit board 41a, the terminal pin 27 of the sensor module 2 for the measurement of the amount of generated ammonia is connected.

As shown in Figs. 1 and 4, a covering material 2d is attached to the waterproof case 4 in such a way as to enclose the vessel main body 20A that is projected outside the waterproof case. This covering material 2d provides a sample solution guiding path 24, which goes along a region adjacent to the outside of the vessel main body 20A and extends in the up and down directions with the both ends open.

The waterproof wire 5 extends upward from the waterproof case 4 through the upper ceiling of the tank 100, whereby the end of the wire is positioned outside the tank. A connector 51 is attached to the end of the waterproof wire 5 in order to connect to an outer circuit. The waterproof wire 5 contains a wire for supplying electricity to the electric source circuit unit 41 and a wire through which an output signal from the sensor module 2 for measuring the amount of generated ammonia is fed via the circuit board 41a.

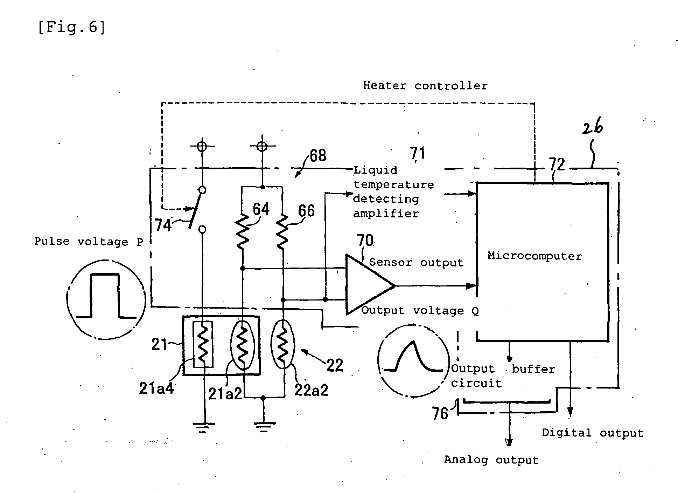

In Fig.6, a circuit for the measurement of the amount of generated ammonia in the present embodiment is shown. A bridge circuit 68 (a liquid detecting circuit) is formed by the temperature-sensitive element 21a2 of the indirectly-heated liquid detecting unit 21, the temperature-sensitive element 22a2 of the liquid temperature detecting unit 22, and two resistor elements 64 and 66. An output from this bridge circuit 68 is entered into a differential amplifier 70, and the amplified output (also referred to as liquid detecting circuit output or sensor output) is entered into a microcomputer 72 that constitutes operation part, via an A/D converter which is not shown in the figure.

Also, an output value which corresponds to the temperature of the sample solution, is fed from the temperature-sensitive element 22a2 of the liquid temperature detecting unit 22 and is entered into the microcomputer 72 via a liquid temperature detecting amplifier 71. On the other hand, the microcomputer 72 outputs a heater controlling signal, based on which a switch 74 is turned on or off. The switch is placed on a circuit that energizes the heating element 21a4 of the indirectly-heated liquid detecting unit 21.

In this embodiment, the part enclosed by a dashed-dotted line in Fig.6 is incorporated in the application specific IC 26.

In Fig.6, the switch 74 is described to make or break a circuit by being opened or closed for simplicity. However, plural voltage application paths may be incorporated in the application specific IC 26 so that different voltages can be applied and thus one may choose an appropriate voltage application path for the heater control. Such structure broadens the range of options for choosing characteristics of the heating element 21a4 of the liquid detecting unit 21. That is, an optimum voltage for the measurement can be applied in accordance with the characteristics of the heating element 21a4. Also, since plural voltages which are different from each other can be applied for the heater control, the range of options for the kinds of the liquids to be measured can be broadened.

The resistor elements 64 and 66 in Fig. 6 are described as having a constant resistance for simplicity. However, in the application specific IC 26, these resistor elements 64 and 66 may have variable resistance values so that the resistance values of the resistor elements 64 and 66 may be changed appropriately in the measurement. By the same token, the application specific IC 26 may be designed so that the characteristics of the differential amplifier 70 and the liquid temperature detecting amplifier 71 can be changed appropriately in the measurement.

Such structure facilitates the optimum setting of the characteristics of the liquid detecting circuit and reduces the variance of measurement characteristics that is caused by the variance in manufacturing the liquid detecting units 21 and the liquid temperature detecting units 22 and the variance in manufacturing the application specific integrated circuits 26, thus leading to an improvement of production yield.

In the following, operation for the measurement of the amount of generated ammonia in the present embodiment will be explained.

When the tank 100 is filled with a sample solution US, the sample solution guiding path 24, which is formed by the covering material 2d that covers the sensor module 2 for the measurement of the amount of generated ammonia, is also filled with the solution US. The solution US does not substantially flow in the sample solution guiding path 24 as well as in the tank 100.

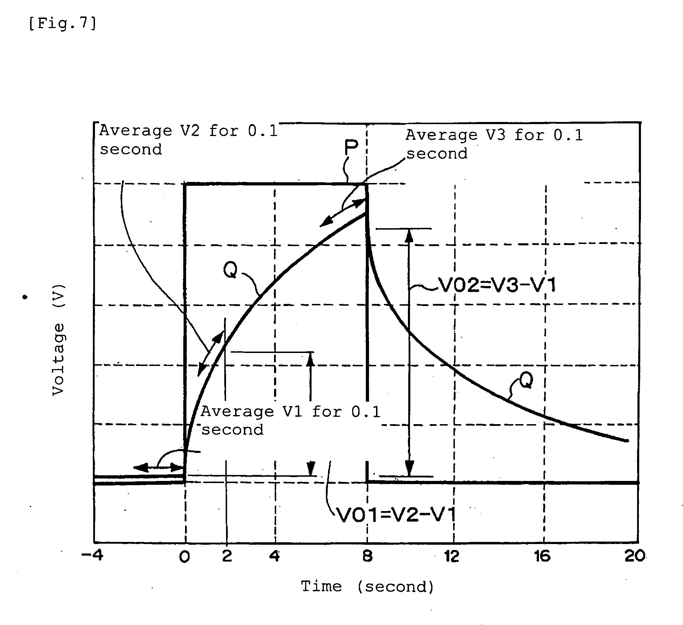

In response to a heater control signal fed from the microcomputer 72 to the switch 74, the switch 74 is closed for a predetermined time (8 seconds for example) to apply a single pulse voltage P with a certain height (10V for example) to the heating element 21a4, whereby the heating element is heated. The output voltage Q at this time (the sensor output) from the differential amplifier 70 increases gradually during the voltage application to the heating element 21a4, and then decreases gradually after termination of the voltage application to the heating element 21a4, as shown in Fig.7.

In the microcomputer 72, as shown in Fig.7, the sensor outputs are sampled predetermined times (256 times for example) for a predetermined period (0.1 second for example) before initiating the voltage application to the heating element 21a4, and an average value thereof is calculated to obtain an average initial voltage value V1. This average initial voltage value V1 corresponds to an initial temperature of the temperature-sensitive element 21a2.

Also, as shown in Fig.7, the sensor outputs are sampled predetermined times (256 times for example) at the passage of a first period (in more detail, immediately before the passage of the first period), which is a relatively short period from the start of the voltage application to the heating element (for example, the first period is 1/2 or less of the application period of the single pulse and is 0.5 to 3 seconds; it is 2 seconds in Fig.7). An average value thereof is calculated to obtain an average first voltage value V2. This average first voltage value V2 corresponds to a first temperature of the temperature-sensitive element 21a2 at the passage of the first period from the initiation of single pulse application. Then, V01 (= V2 - V1), the difference between the average initial voltage value V1 and the average first voltage value V2, is obtained as a thermal conductivity-dependent voltage value.

Also, as shown in Fig.7, the sensor outputs are sampled predetermined times (256 times for example) at the passage of a second period (in more detail, immediately before the passage of the second period), which is a relatively long period from the start of the voltage application to the heating element (for example, the second period is equal to the application period of the single pulse; it is 8 seconds in Fig. 7). An average value thereof is calculated to obtain an average second voltage value V3. This average second voltage value V3 corresponds to a second temperature of the temperature-sensitive element 21a2 at the passage of the second period from the initiation of single pulse application. Then, V02 (= V3 - V1), the difference between the average initial voltage value V1 and the average second voltage value V3 is obtained as a kinetic viscosity-dependent voltage value.

In the meanwhile, part of the heat generated at the heating element 21a4 by the above-mentioned application of the single pulse voltage is transferred to the temperature-sensitive element 21a2 via the sample solution. Herein, the heat is transferred mainly in two ways depending on the time period from the initiation of the pulse application. In the first stage which is within a relatively short period of the start of the pulse application (3 seconds for example, in particular 2 seconds), the heat is dominantly transferred by the thermal conductance (for this reason, the thermal conductivity-dependent voltage value V01 is mainly affected by the thermal conductivity of the solution).

In contrast, in the second stage after the first stage, the heat transfer is mainly influenced by the natural convection (for this reason, the kinetic viscosity-dependent voltage value V02 is mainly affected by the kinetic viscosity of the solution). This is because, in the second stage, the heating in the first stage produces natural convection of the sample solution, and the heat is increasingly transferred by the natural convection.

The thermal conductivity-dependent voltage value V01 and the kinetic viscosity-dependent voltage value V02 change with the concentrations of urea and ammonium formate in the sample solution US.

In the present invention, the amount of ammonia generated from the mixture solution is measured by measuring the concentrations of urea and ammonium formate. For the measurement, the present invention utilizes the fact that the relationship between the thermal conductivity-dependent voltage value V01 and the kinetic viscosity-dependent voltage value V02 varies depending on the concentrations of urea and ammonium formate in the mixture solution, as mentioned above. In more detail, the thermal conductivity-dependent voltage value V01 and the kinetic viscosity-dependent voltage value V02 are affected by different physical properties of the solution, namely, by the thermal conductivity and the kinetic viscosity respectively. And the concentrations of urea and ammonium formate in the mixture solution can be determined because the relationship of the above voltage values changes with the concentrations.

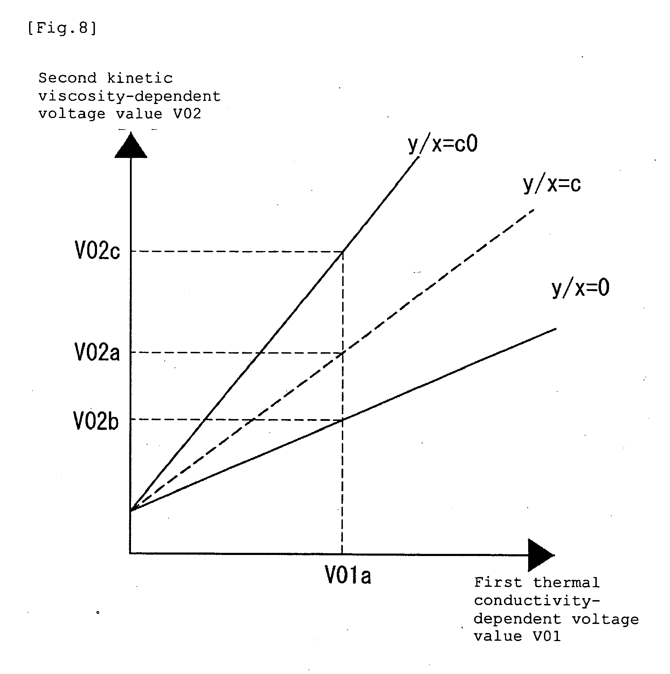

The present embodiment will be specifically described below. First calibration curves are previously prepared which show relationships between the thermal conductivity-dependent voltage value V01 and the kinetic viscosity-dependent voltage value V02, with respect to several aqueous urea solutions (referential aqueous urea solutions) in which Y/X = 0 (Y is the ammonium formate concentration wt% and X is the urea concentration wt%, wherein the ammonium formate concentration is 0% and the urea concentration is known). Second calibration curves are previously prepared which show relationships between the thermal conductivity-dependent voltage value V01 and the kinetic viscosity-dependent voltage value V02, with respect to several mixture solutions in which Y/X = c0 (constant) (Y is the ammonium formate concentration wt% and X is the urea concentration wt%). These calibration curves are memorized in memory means of the microcomputer 72. Fig. 8 shows examples of the first and the second calibration curves.

Here, assume that a thermal conductivity-dependent voltage value of V01a and a kinetic viscosity-dependent voltage value of V02a are obtained with a sample solution US which is a mixture solution with a urea concentration of Xa% and an ammonium formate concentration of Ya%.

If the solution contains only urea (aqueous urea solution), the kinetic viscosity-dependent voltage value should be V02b when the thermal conductivity-dependent voltage value is V01a, as shown in Fig.8. Thus, the fact that the thermal conductivity-dependent voltage value V01 and the kinetic viscosity-dependent voltage value V02 do not cross on the first calibration curve means that the solution contains not only urea but also ammonium formate.

The first and the second calibration curves obtained in advance show that, when the thermal conductivity-dependent voltage value is V01a, the tentative kinetic viscosity-dependent voltage value for Y/X = 0 is V02b, and the tentative kinetic viscosity-dependent voltage value for Y/X = c0 is V02c.

Then, calculation is performed on a pro-rata basis to determine the value c of Y/X = c that provides a thermal conductivity-dependent voltage value of V01a and a kinetic viscosity-dependent voltage value of V02a. Namely, the value is obtained from the following equation:

$$c = c0(V02a - V02b)/V02c.$$

Meanwhile, because the first and the second calibration curves in Fig.8 change with the liquid temperature, it is necessary to obtain calibration curves at plural liquid temperatures in advance, memorize the curves in the memory means of the microcomputer 72, and appropriately change the calibration curves depending on the liquid temperature.

When Y/X = c is confirmed, the value X at which the thermal conductivity-dependent voltage value V01 is V01a is determined from a previously memorized calibration curve (comparison curve) that shows a relation between the thermal conductivity-dependent voltage value V01 and the urea concentration X for Y/X = c. Then, the value Y can be determined based on the value X determined.

Alternatively, a calibration curve (comparison curve) between the thermal conductivity-dependent voltage value V01 and the urea concentration X for Y/X = c may be interpolated from previously memorized calibration curves for several c values.

Then, the amount of generated ammonia per unit weight of the sample solution US by the following equation with 4 parameters of the urea concentration X wt%, the ammonium formate concentration Y wt%, the amount of ammonia A generated from per unit weight of urea, and the amount of ammonia B generated from per unit weight of ammonium formate.

$$\text{The amount of generated ammonia } G = X / 100 \text{ x } A + Y / 100$$

$$\text{x } B.$$

The amount of generated ammonia calculated here is a maxima. The generation efficiency changes according to the condition as the temperature etc., consequently the amount of generated ammonia might become fewer than the calculated value.

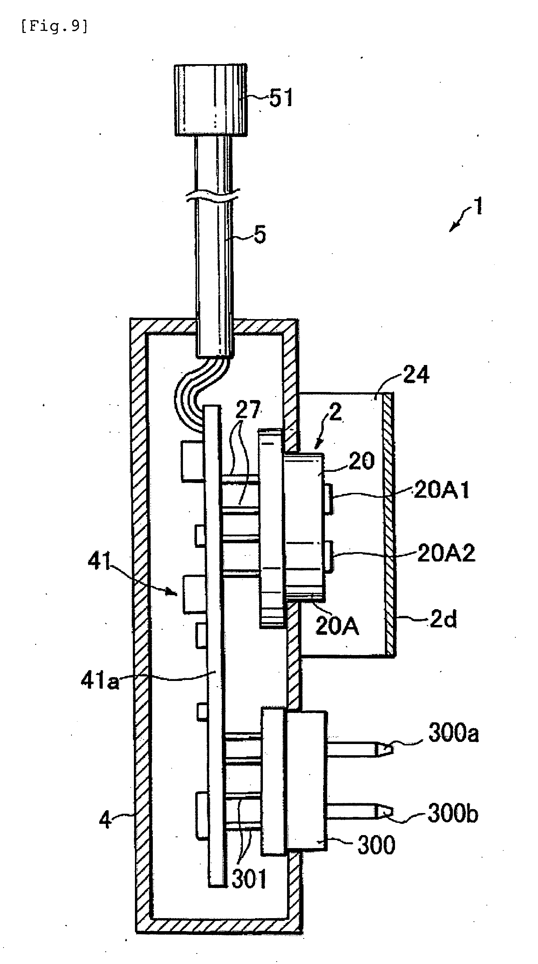

Next, a second embodiment of the present invention will be explained. In the present embodiment, as shown in Fig.9, a differential pressure sensor 300 is installed in addition to a sensor module 2 for measurement of the amount of generated ammonia. Here, the differential pressure sensor 300 may be conventional.

Meanwhile, an apparatus 1 for measurement of the amount of generated ammonia as shown in Fig.9 has basically the same constitution as that of the apparatus 1 for measurement of the amount of generated ammonia in the embodiment shown in Figs. 1 to 8. Thus, the same reference numerals are assigned for the corresponding constitutional materials and a detail explanation therefor will be omitted.

As shown in Fig. 9, a terminal pin 301 of the differential pressure sensor 300 is connected to a circuit of the circuit board 41a.

When the liquid pressure at a first entrance 300a of the differential pressure sensor 300 is p1, the liquid pressure at a second entrance 300b is p2, the height difference between the first entrance 300a and the second entrance 300b is L, and the density of the sample solution is $\rho$, a density-dependent voltage value V03, namely, an electrical output dependent on p can be measured with the differential pressure sensor 300, since there is a relationship of p1 - p2 = $\rho$ x L.

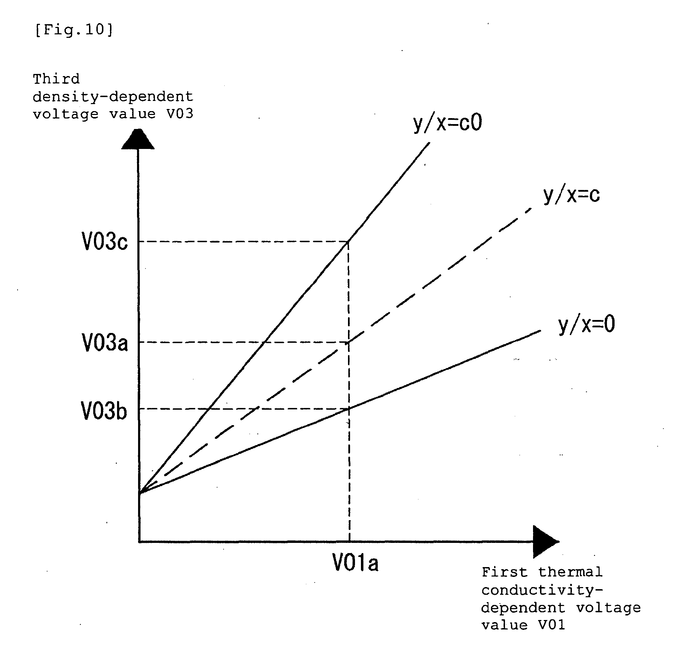

The present embodiment will be specifically described below. The thermal conductivity-dependent voltage value V01 is measured with the sensor module 2 with respect to several aqueous urea solutions (referential aqueous urea solutions) in which Y/X = 0 (Y is the ammonium formate concentration wt% and X is the urea concentration wt%, wherein the ammonium formate concentration is 0% and the urea concentration is known). Then, third calibration curves are previously prepared which show relationships between the thermal conductivity-dependent voltage value V01 and the density-dependent voltage value V03. Similarly, the thermal conductivity-dependent voltage value V01 is measured with the sensor module 2 with respect to several mixture solutions in which Y/X = c0 (constant) (Y is the ammonium formate concentration wt% and X is the urea concentration wt%) . And fourth calibration curves are previously prepared which show relationships between the thermal conductivity-dependent voltage value V01 and the density-dependent voltage value V03 . These calibration curves are memorized in memory means of the microcomputer 72. Fig. 10 shows examples of the third and the fourth calibration curves.

Meanwhile, since it is not necessary to obtain the kinetic viscosity-dependent voltage value V02 in the present embodiment, the application of the pulse voltage may' be terminated at the passage of the first period, which is a relatively short period from the start of the voltage application to the heating element. That is, the first period may be taken as the period of the pulse voltage application. Thus, the measurement time can be shortened.

Here, assume that a thermal conductivity-dependent voltage value of v01a and a density-dependent voltage value of V03a are obtained with a sample solution US that is a mixture solution with a urea concentration of Xa% and an ammonium formate concentration of Ya%.

If the solution contains only urea (aqueous urea solution), the density-dependent voltage value should be V03b when the thermal conductivity-dependent voltage value is V01a as shown in Fig.10. Therefore, the fact that the thermal conductivity-dependent voltage value V01 and the density-dependent voltage value V03 do not cross on the third calibration curve means that the solution contains not only urea but also ammonium formate.

The third and the fourth calibration curves obtained in advance show that, when the thermal conductivity-dependent voltage value is V01a, the density-dependent voltage value for Y/X = 0 is V03b, and the density-dependent voltage value for Y/X = c0 is V03c.

Calculation is performed on a pro-rata basis to determine the value c of Y/X = c that provides a thermal conductivity-dependent voltage value of V01a and a density-dependent voltage value of V03a. Namely, the value is obtained from the following equation.

$$c = c0(V03a - V03b)/V03c$$

.

Meanwhile, because the third and the fourth calibration curves in Fig.10 change with the liquid temperature, it is necessary to obtain calibration curves at plural liquid temperatures in advance, and appropriately change the calibration curves depending on the liquid temperature.

From Y/X = c, the values of X and Y are determined at which the first voltage value V01 is V01a, and then the amount of generated ammonia can be calculated in a similar manner as mentioned above.

In the above, preferable embodiments of the present invention have been explained, but the present invention is not restricted by these embodiments, and the pulse voltage, sampling numbers, and the like may be appropriately changed.

Although the above embodiments explain the invention applied to a tank for NOx decomposition that constitutes a cleaning system for exhaust gases mounted on an automobile, the invention can be also applied in a similar manner to NOx decomposition systems for a ship, a rail vehicle, a two-wheeled motor vehicle, a construction machine, a power generator and the like that generate NOx. Various modifications are possible as long as they do not impair the obj ect of the present invention.

| | |
|---|---|
| 1 | Apparatus for measuring the amount of generated ammonia |
| 2 | Sensor module for measurement of the amount of generated ammonia |
| 2d | Covering material |
| 4 | Waterproof case |
| 5 | Waterproof wire |
| 20 | Vessel |
| 20A | Vessel main body part |
| 20A1 | Blister part |
| 20B | Vessel lid |
| 21 | Liquid detecting unit |
| 21a | Thin film chip for liquid detection |
| 21a1 | Chip substrate |
| 21a2 | Temperature-sensitive element for liquid detection |
| 21a3 | Interlayer insulating film |
| 21a4 | Heating element |
| 21a5 | Heating element electrode |
| 21a6 | Protection film |
| 21a7 | Electrode pad |
| 21d | Bonding wire |
| 21e | Outer electrode terminal |
| 22 | Liquid temperature detecting unit |
| 22a2 | Temperature-sensitive element for liquid temperature Detection |
| 22e | Outer electrode terminal |
| 23 | Synthetic resin mold |
| 24 | Sample solution guiding path |
| 25 | Liquid detecting circuit board |
| 26 | Application specific IC |

| 27 | Terminal pin |
| 41 | Electric source circuit |
| 41a | Circuit board |
| 51 | Connector |
| 64 | Resistor element |
| 66 | Resistor element |
| 68 | Bridge circuit |
| 70 | Differential amplifier |
| 71 | Liquid temperature detecting amplifier |
| 72 | Microcomputer |
| 74 | Switch |
| 100 | Tank |
| 101 | Wall material |
| 300 | Differential pressure sensor |
| 300a | The first guiding path |
| 300b | The second guiding path |
| 301 | Terminal pin |
| US | Sample solution |

**Claims**

1. A method for measuring an amount of ammonia generated from a sample solution (US) being an aqueous urea solution, an aqueous ammonium formate solution, or an aqueous mixture thereof, comprising:

   providing a sensor (2) comprising a heating element (21a4) and a temperature-sensitive element (21a2) placed near the heating element (21a4);
   applying a pulse voltage to the heating element (21a4) for a predetermined time to heat the sample solution (US) using the heating element (21a4);
   measuring a thermal conductivity-dependent output value that is an electrical output from the temperature-sensitive element (21a2) and depends on thermal conductivity of the sample solution (US), and a kinetic viscosity-dependent output value that is an electrical output from the temperature-sensitive element (21a4) and depends on kinetic viscosity of the sample solution (US), the electrical outputs being dependent on electric resistivity of the temperature-sensitive element (21a2);
   calculating a urea concentration X wt% and an ammonium formate concentration Y wt% in the sample solution (US) from a relationship between the thermal conductivity-dependent output value and the kinetic viscosity-dependent output value;
   determining the amount of generated ammonia per unit weight of the sample solution (US) by using the following equation calculated from an amount of generated ammonia A per unit weight of the urea and an amount of generated ammonia B per unit weight of the ammonium formate and their concentrations:

```
the amount of generated ammonia = X / 100 x A + Y / 100
x B.
```

2. The method for measuring an amount of generated ammonia according to claim 1, wherein the urea concentration X wt% and the ammonium formate concentration Y wt% in the sample solution (US) are calculated from the thermal conductivity-dependent output value and the kinetic viscosity-dependent output value of the sample solution (US) that are obtained based on previously memorized calibration curve data that indicate correlations between a thermal conductivity-dependent output value and a kinetic viscosity-dependent output value of predetermined reference solutions.

3. The method for measuring an amount of generated ammonia according to claim 2, wherein the urea concentration X wt% and the ammonium formate concentration Y wt% are obtained' by determining at least two tentative kinetic viscosity-dependent output values to which the thermal conductivity-dependent output value of the sample solution corresponds, based on at least two pieces of the calibration curve data; and performing calculation on a pro-rata basis using the at least two tentative kinetic viscosity-dependent output values to determine the urea concentration and the ammonium formate concentration at which the kinetic viscosity-dependent output value is obtained.

4. The method for measuring an amount of generated ammonia according to claim 3, wherein

the sample solution (US) has a urea concentration of X wt% and an ammonium formate concentration of Y wt% and gives a thermal conductivity-dependent output value of V01 and a kinetic viscosity-dependent output value of V02;

first calibration curve data are provided in which a ratio of a urea concentration Xb wt% to an ammonium formate concentration Yb wt%, namely $cb = Yb/Xb$, is constant;

second calibration curve data are provided in which a ratio of a urea concentration Xc wt% to an ammonium formate concentration Yc wt%, namely $cc = Yc/Xc$, is constant and larger than the ratio cb;

a first tentative kinetic viscosity-dependent output value V02b and a second tentative kinetic viscosity-dependent output value V02c are obtained to which the thermal conductivity-dependent output value V01 corresponds, based on the first and second calibration curve data;

a ratio of the urea concentration X wt% to the ammonium formate concentration Y wt%, namely $c = Y/X$, is calculated from the formula: $c = cc(V02-V02b)/V02c$ in which V02b is the first tentative kinetic viscosity-dependent output value and V02c is the second tentative kinetic viscosity-dependent output value; and

the urea concentration X wt% and the ammonium formate concentration Y wt% are calculated based on calibration curve data corresponding to $Y/X = c$ that is selected from among the previously memorized calibration curve data.

5. The method for measuring an amount of generated ammonia according to claim 4, wherein the calibration curve data corresponding to $Y/X = c$ is interpolated from the previously memorized calibration curve data.

6. The method for measuring an amount of generated ammonia according to any one of claims 1 to 5, wherein the kinetic viscosity-dependent output value is an electrical output from the temperature-sensitive element (21a2) that is measured from initiation of the application of the pulse voltage to the passage of a second period and the second period is equal to a period of the application of the pulse voltage.

7. The method for measuring an amount of generated ammonia according to claim 6, wherein the period of the application of the pulse voltage is 5 to 30 seconds.

8. The method for measuring an amount of generated ammonia according to any one of claims 1 to 7, wherein the thermal conductivity-dependent output value is an electrical output from the temperature-sensitive element (21a2) that is measured from initiation of the application of the pulse voltage to the passage of a first period and the first period is 1/2 or less of the period of the application of the pulse voltage.

9. The method for measuring an amount of generated ammonia according to claim 8, wherein the first period is 0.5 to 3 seconds.

10. The method for measuring an amount of generated ammonia according to any one of claims 2 to 9, wherein at least one of the previously memorized predetermined reference solutions is an aqueous urea solution having an ammonium formate concentration of 0%.

11. A method for measuring an amount of ammonia generated from a sample solution (US) being an aqueous urea solution, an aqueous ammonium formate solution, or an aqueous mixture thereof, comprising:

providing a sensor (2) comprising a heating element (21a4) and a temperature-sensitive element (21a2) placed near the heating element (21a4);

applying a pulse voltage to the heating element (21a4) for a predetermined time to heat the sample solution (US) using the heating element (21a4);

measuring a thermal conductivity-dependent output value that is an electrical output from the temperature-sensitive element (21a2) and depends on thermal conductivity of the sample solution (US), the electrical output being dependent on electric resistivity of the temperature-sensitive element (21a2), and measuring a density-dependent output value that is an electrical output dependent on density of the sample solution with a differential pressure sensor (300);

calculating a urea concentration X wt% and an ammonium formate concentration Y wt% in the sample solution (US) from a relationship between the thermal conductivity-dependent output value and the density-dependent output value;

determining the amount of generated ammonia per unit weight of the sample solution (US) by using the following equation calculated from an amount of generated ammonia A per unit weight of the urea and an amount of generated ammonia B per unit weight of the ammonium formate and their concentrations:

```
the amount of generated ammonia = X / 100 x A + Y / 100

x B.
```

12. The method for measuring an amount of generated ammonia according to claim 11, wherein the urea concentration X wt% and the ammonium formate concentration Y wt% in the sample solution (US) are calculated from the thermal conductivity-dependent output value and the density-dependent output value of the sample solution (US) that are obtained based on previously memorized calibration curve data that indicate correlations between a thermal conductivity-dependent output value and a density-dependent output value of predetermined reference solutions.

13. The method for measuring an amount of generated ammonia according to claim 12, wherein the urea concentration X wt% and the ammonium formate concentration Y wt% are obtained by determining at least two tentative density-dependent output values to which the thermal conductivity-dependent output value of the sample solution (US) corresponds, based on at least two pieces of the calibration curve data; and performing calculation on a pro-rata basis using the at least two tentative density-dependent output values to determine the urea concentration and the ammonium formate concentration at which the density-dependent output value of the sample solution (US) is obtained.

14. The method for measuring an amount of generated ammonia according to claim 13, wherein

the sample solution (US) has a urea concentration of X wt% and an ammonium formate concentration of Y wt% and gives a thermal conductivity-dependent output value of V01 and a density-dependent output value of V03;

third calibration curve data are provided in which a ratio of a urea concentration Xb wt% to an ammonium formate concentration Yb wt%, namely cb = Yb/Xb, is constant;

fourth calibration curve data are provided in which a ratio of a urea concentration Xc wt% to an ammonium formate concentration Yc wt%, namely cc = Yc/Xc, is constant and larger than the ratio cb;

a first tentative density-dependent output value V03b and a second tentative density-dependent output value V03c are obtained to which the thermal conductivity-dependent output value V01 corresponds, based on the third and fourth calibration curve data;

a ratio of the urea concentration X wt% to the ammonium formate concentration Y wt%, namely c = Y/X, is calculated from the formula: c = cc (V03-V03b) /V03c in which V03b is the first tentative density-dependent output value and V03c is the second tentative density-dependent output value; and

the urea concentration X wt% and the ammonium formate concentration Y wt% are calculated based on calibration curve data corresponding to Y/X = c that is selected from among the previously memorized calibration curve data.

15. The method for measuring an amount of generated ammonia according to claim 14, wherein the calibration curve data corresponding to Y/X = c is interpolated from the previously memorized calibration curve data.

16. The method for measuring an amount of generated ammonia according to any one of claims 11 to 15, wherein the thermal conductivity-dependent output value is an electrical output from the temperature-sensitive element (21a2) that is measured from initiation of the application of the pulse voltage to the passage of a first period and the first period is 1/2 or less of the period of the application of the pulse voltage.

17. The method for measuring an amount of generated ammonia according to claim 16, wherein the first period is 0.5 to 3 seconds.

18. The method for measuring an amount of generated ammonia according to any one of claims 12 to 17, wherein at least one of the previously memorized predetermined reference solutions is an aqueous urea solution having an ammonium formate concentration of 0%.

19. An apparatus (1) for measuring an amount of ammonia generated from a sample solution (US) being an aqueous urea solution, an aqueous ammonium formate solution, or an aqueous mixture thereof, comprising:

a sensor (2) comprising a heating element (21a4) and a temperature-sensitive element (21a2) placed near the heating element (21a4); and being configured to:

apply a pulse voltage to the heating element (21a4) for a predetermined time to heat the sample solution (US) using the heating element;
measure a thermal conductivity-dependent output value that is an electrical output from the temperature-sensitive element (21a2) and depends on thermal conductivity of the sample solution (US), and a kinetic viscosity-dependent output value that is an electrical output from the temperature-sensitive element (21a2) and depends on kinetic viscosity of the sample solution, the electrical outputs being dependent on electric resistivity of the temperature-sensitive element (21a2);
calculate a urea concentration X wt% and an ammonium formate concentration Y wt% in the sample solution (US) from a relationship between the thermal conductivity-dependent output value and the kinetic viscosity-dependent output value;
determine the amount of generated ammonia per unit weight of the sample solution (US) by using the following equation calculated from an amount of generated ammonia A per unit weight of the urea and an amount of generated ammonia B per unit weight of the ammonium formate and their concentrations:

```
the amount of generated ammonia = X / 100 x A + Y / 100
```

```
x B.
```

20. The apparatus (1) for measuring an amount of generated ammonia according to claim 19, wherein the urea concentration X wt% and the ammonium formate concentration Y wt% in the sample solution (US) are calculated from the thermal conductivity-dependent output value and the kinetic viscosity-dependent output value of the sample solution (US) that are obtained based on previously memorized calibration curve data that indicate correlations between a thermal conductivity-dependent output value and a kinetic viscosity-dependent output value of predetermined reference solutions.

21. The apparatus (1) for measuring an amount of generated ammonia according to claim 20, wherein the urea concentration X wt% and the ammonium formate concentration Y wt% are obtained by determining at least two tentative kinetic viscosity-dependent output values to which the thermal conductivity-dependent output value of the sample solution (US) corresponds, based on at least two pieces of the calibration curve data; and performing calculation on a pro-rata basis using the at least two tentative kinetic viscosity-dependent output values to determine the urea concentration and the ammonium formate concentration at which the kinetic viscosity-dependent output value is obtained.

22. The apparatus (1) for measuring an amount of generated ammonia according to claim 21, wherein

the sample solution (US) has a urea concentration of X wt% and an ammonium formate concentration of Y wt% and gives a thermal conductivity-dependent output value of V01 and a kinetic viscosity-dependent output value of V02;
first calibration curve data are provided in which a ratio of a urea concentration Xb wt% to an ammonium formate concentration Yb wt%, namely cb = Yb/Xb, is constant;
second calibration curve data are provided in which a ratio of a urea concentration Xc wt% to an ammonium formate concentration Yc wt%, namely cc = Yc/Xc, is constant and larger than the ratio cb;
a first tentative kinetic viscosity-dependent output value V02b and a second tentative kinetic viscosity-dependent output value V02c are obtained to which the thermal conductivity-dependent output value V01 corresponds, based on the first and second calibration curve data;
a ratio of the urea concentration X wt% to the ammonium formate concentration Y wt%, namely c = Y/X, is calculated from the formula: c = cc (V02-V02b)/V02c in which V02b is the first tentative kinetic viscosity-dependent output value and V02c is the second tentative kinetic viscosity-dependent output value; and
the urea concentration X wt% and the ammonium formate concentration Y wt% are calculated based on calibration curve data corresponding to Y/X = c that is selected from among the previously memorized calibration curve data.

23. The apparatus (1) for measuring an amount of generated ammonia according to claim 22, wherein the calibration curve data corresponding to Y/X = c is interpolated from the previously memorized calibration curve data.

24. The apparatus (1) for measuring an amount of generated ammonia according to any one of claims 19 to 23, wherein the kinetic viscosity-dependent output value is an electrical output from the temperature-sensitive element (21a2) that is measured from initiation of the application of the pulse voltage to the passage of a second period and the second period is equal to a period of the application of the pulse voltage.

25. The apparatus (1) for measuring an amount of generated ammonia according to claim 24, wherein the period of the application of the pulse voltage is 5 to 30 seconds.

26. The apparatus (1) for measuring an amount of generated ammonia according to any one of claims 19 to 25, wherein the thermal conductivity-dependent output value is an electrical output from the temperature-sensitive element (21a2) that is measured from initiation of the application of the pulse voltage to the passage of a first period and the first period is 1/2 or less of the period of the application of the pulse voltage.

27. The apparatus (1) for measuring an amount of generated ammonia according to claim 26, wherein the first period is 0.5 to 3 seconds.

28. The apparatus (1) for measuring an amount of generated ammonia according to any one of claims 20 to 27, wherein at least one of the previously memorized predetermined reference solutions is an aqueous urea solution having an ammonium formate concentration of 0%.

29. An apparatus (1) for measuring an amount of ammonia generated from a sample solution (US) being an aqueous urea solution, an aqueous ammonium formate solution, or an aqueous mixture thereof, comprising:

a sensor (2) comprising a heating element (21a4) and a temperature-sensitive element (21a2) placed near the heating element (21a4); and being configured to:

apply a pulse voltage to the heating element (21a4) for a predetermined time to heat the sample solution (US) using the heating element (21a4);
measure a thermal conductivity-dependent output value with the sensor (2), which output value is an electrical output from the temperature-sensitive element (21a2) and depends on thermal conductivity of the sample solution (US), the electrical output being dependent on electric resistivity of the temperature-sensitive element (21a2), and measure a density-dependent output value with a differential pressure sensor (300), which output value is an electrical output dependent on density of the sample solution (US);
calculate a urea concentration X wt% and an ammonium formate concentration Y wt% in the sample solution (US) from a relationship between the thermal conductivity-dependent output value and the density-dependent output value;
determine the amount of generated ammonia per unit weight of the sample solution (US) by using the following equation calculated from an amount of generated ammonia A per unit weight of the urea and an amount of generated ammonia B per unit weight of the ammonium formate and their concentrations:

```
the amount of generated ammonia = X / 100 x A + Y / 100
```

```
 x B.
```

30. The apparatus (1) for measuring an amount of generated ammonia according to claim 29, wherein the urea concentration X wt% and the ammonium formate concentration Y wt% in the sample solution (US) are calculated from the thermal conductivity-dependent output value and the density-dependent output value of the sample solution that are obtained based on previously memorized calibration curve data that indicate correlations between a thermal conductivity-dependent output value and a density-dependent output value of predetermined reference solutions.

31. The apparatus (1) for measuring an amount of generated ammonia according to claim 30, wherein the urea concentration X wt% and the ammonium formate concentration Y wt% are obtained by determining at least two tentative density-dependent output values to which the thermal conductivity-dependent output value of the sample solution (US) corresponds, based on at least two pieces of the calibration curve data; and performing calculation on a pro-rata basis using the at least two tentative density-dependent output values to determine the urea concentration and the ammonium formate concentration at which the density-dependent output value of the sample solution (US) is obtained.

32. The apparatus (1) for measuring an amount of generated ammonia according to claim 31, wherein

the sample solution (US) has a urea concentration of X wt% and an ammonium formate concentration of Y wt% and gives a thermal conductivity-dependent output value of V01 and a density-dependent output value of V03;
third calibration curve data are provided in which a ratio of a urea concentration Xb wt% to an ammonium formate concentration Yb wt%, namely cb = Yb/Xb, is constant;
fourth calibration curve data are provided in which a ratio of a urea concentration Xc wt% to an ammonium formate concentration Yc wt%, namely cc = Yc/Xc, is constant and larger than the ratio cb;
a first tentative density-dependent output value V03b and a second tentative density-dependent output value V03c are obtained to which the thermal conductivity-dependent output value V01 corresponds, based on the third and fourth calibration curve data;
a ratio of the urea concentration X wt% to the ammonium formate concentration Y wt%, namely c = Y/X, is calculated from the formula: c = cc (V03-V03b) /V03c in which V03b is the first tentative density-dependent output value and V03c is the second tentative density-dependent output value; and
the urea concentration X wt% and the ammonium formate concentration Y wt% are calculated based on calibration curve data corresponding to Y/X = c that is selected from among the previously memorized calibration curve data.

33. The apparatus (1) for measuring an amount of generated ammonia according to claim 32, wherein the calibration curve data corresponding to Y/x = c is interpolated from the previously memorized calibration curve data.

34. The apparatus (1) for measuring an amount of generated ammonia according to any one of claims 29 to 33, wherein the thermal conductivity-dependent output value is an electrical output from the temperature-sensitive element (21a2) that is measured from initiation of the application of the pulse voltage to the passage of a first period and the first period is 1/2 or less of the period of the application of the pulse voltage.

35. The apparatus (1) for measuring an amount of generated ammonia according to claim 34, wherein the first period is 0.5 to 3 seconds.

36. The apparatus (1) for measuring an amount of generated ammonia according to any one of claims 30 to 35, wherein at least one of the previously memorized predetermined reference solutions is an aqueous urea solution having an ammonium formate concentration of 0%.

[Fig.1]

[Fig.2]

[Fig.3]

[Fig.4]

[Fig.5]

[Fig.6]

[Fig.7]

[Fig.8]

[Fig.9]

[Fig.10]

Third
density-dependent
voltage value V03

V03c

V03a

V03b

y/x=c0

y/x=c

y/x=0

V01a

First thermal
conductivity-
dependent voltage
value V01